# EUROPEAN PATENT SPECIFICATION

(11) **EP 1 695 984 B1**
(45) Date of publication and mention of the grant of the patent: **06.05.2015**
(21) Application number: 06447025.5
(22) Date of filing: 20.02.2006
(51) Int. Cl.: C07K 16/18, A61L 27/34

(54) **Use of a reference material for assaying d-dimer**
Verwendung eines Referenzmaterials zum Untersuchen von D-Dimeren
Utilisation d'un matériau de référence pour l'analyse de dimères D

(30) Priority: 25.02.2005 JP 2005051524
(43) Date of publication of application: 30.08.2006
(73) Proprietor: SYSMEX CORPORATION, Kobe-shi, Hyogo 651-0073 (JP)
(72) Inventor: Okuda, Masahiro, c/o Sysmex Corporation, Kobe-shi, Hyogo 651-0073 (JP); Takeshita, Hiroki, c/o Sysmex Corporation, kobe-shi, Hyogo 651-0073 (JP)
(74) Representative: De Clercq, Ann G. Y.

(56) References cited:
- WO-A-90/14102
- WO-A-03/000736
- US-A- 5 693 098
- FUJITA M ET AL: "Characterization of nattokinase-degraded products from human fibrinogen or cross-linked fibrin" FIBRINOLYSIS, CHURCHILL LIVINGSTONE, LONDON, GB, vol. 9, no. 3, May 1995 (1995-05), pages 157-164, XP004919984 ISSN: 0268-9499

## Description

### TECHNICAL FIELD

The present invention relates to the use of a reference material for assaying D-dimer, as defined in claim 1.

### BACKGROUND

It is known that in the field of clinical laboratory testing, in particular, blood coagulation fibrinolytic testing, D-dimer is measured. D-dimer is one type of blood coagulation molecular marker, and it is important to measure this when diagnosing various types of thrombosis which enhance the coagulation/fibrinolytic system, and DIC (disseminated intravascular coagulation syndrome), and when gaining a barometer for determining the pathosis of these and judging the effects of curing.

D-dimer is a fibrin degradation product that is gained when a stabilized fibrin which is a polymer that is formed when fibrinogen in blood coagulates due to the effects of thrombin or the like degrades due to enzymes such as plasmin, and is a general term for DD/E fractions and multimers (XDP) having these as a base unit. The multimers of the DD/E fractions include DXD/YY fractions (trimers of DD/E fractions), YXY/DXXD fractions (pentamers of DD/E fractions), DXXD/YXXY fractions (heptamers of DD/E fractions) and the like.

In the field of clinical laboratory testing, generally, a calibration curve is gained using a reference material, and the reliability of testing is enhanced by using a reagent for quality control. In the testing of D-dimer, human plasma is used as a specimen, and therefore, it is desired for the reference material to be used or the responsiveness of the reagent for quality control to be at approximately the same level as that of human plasma. In addition, it is preferable for the concentration of D-dimer in the specimen which is the object to be measured to fall within the range of the calibration point, and therefore, in many cases, the concentration of the reference material for gaining the calibration curve is set so as to be no lower than the upper limit of the normal value, so that specimens that contain a high concentration of D-dimer can also be measured.

It is known that blood plasma that has been taken from a human is conventionally used as it is, as a reference material or a reagent for quality control. Though such reference materials are preferable in that the responsiveness is the same, the concentration of D-dimer is not stable, and this concentration is low and insufficient for gaining a desired calibration curve. Furthermore, a problem arises, such that human originated materials cannot be stably supplied.

Though conventionally, it has been believed that the main component of D-dimer in the plasma of a thrombosis patient is a DD/E fraction of which the molecular weight is approximately 230, 000, it has been becoming clear in recent years that the main component is actually a multimer having a higher molecular weight, such as DXD/YY fractions, YXY/DXXD fractions, or DXXD/YXXY fractions (see Documents 1 and 2). D-dimer reference materials which are gained in accordance with a conventional method for purifying D-dimer fractions (see Documents 3 and 4), however, include a large amount of DD/E fractions, and a reference material for measuring D-dimer which include a large amount of a multimer having a high molecular weight and are appropriate for the clinical behavior in actual thrombosis patients have been desired.

Document 1: Dempfle CE, Zips S, Ergul H, Heene DL; Fivrin Assay Comparative Trial study group. The Fibrin Assay Comparison Trial (FACT): evaluation of 23 quantitative D-dimer assays as basis for the development of D-dimer calibrators. FACT study group. Thromb Haemost. (2001) 85: 671-678

Document 2: Francis CW, Marder VJ, Martin SE; Plasmic degradation of crosslinked fibrin. I. Structural analysis of the particulate clot and identification of new macromolecular-soluble complexes. Blood. 1980 Sep; 56(3): 456-464

Document 3: Gaffney PJ, Edgell T, Creighton-Kempsford LJ, Wheeler S, Tarelli E; Fibrin degradation product (FnDP) assays: analysis of standardization issues and target antigens in plasma. Br J Haematol. 1995 May: 90(1): 187-194

Document 4: Olexa SA, Budzynski AZ; Primary soluble plasmic degradation product of human cross-linked fibrin. Isolation and stoichiometry of the (DD) E complex. Biochemistry. 1979 Mar 20; 18 (6): 991-995

### SUMMARY

The present invention provides the use of a reference material for assaying D-dimer, comprising a fibrin degradation product, wherein no less than 70 % of a total peak area has the molecular weight of no less than 500, 000 in a molecular weight distribution of the fibrin degradation product, measured by gel filtration chromatography. The reference material per se is not part of the present invention.

In the present specification, the term "fibrin degradation product" means degradation product which is obtained by using enzymes act on fibrin and includes multimers such as DD fractions, DD/E fractions, DXD/YY fractions (trimers of DD/E fractions), YXY/DXXD fractions (pentamers of DD/E fractions) and DXXD/YXXY fractions (heptamers of DD/E fractions).

### BRIEF DESCRIPTION OF THE DRAWINGS

Fig. 1 is a chart showing the gel filtration chromatography for a wavelength of 280 nm in the process for fractionation in order to gain a reference material for measuring D-dimer according to the present embodiment;
Fig. 2 is a chart showing the gel filtration chromatography for a wavelength of 280 nm of a reference material for measuring D-dimer according to the present embodiment;
Fig. 3 shows the results of measurement of the amount of D-dimer by means of a latex agglutination method using a reference material for measuring D-dimer according to the present embodiment and plasma from a DIC patient; and
Fig. 4 shows the results of Western blotting of a reference material for measuring D-dimer as used in the present embodiment and that of plasma from a thrombosis patient.

### DESCRIPTION OF THE PREFERRED EMBODIMENTS

A reference material for measuring D-dimer according to the present embodiment is made of fibrin degradation products which are degradation products gained by making enzymes act on fibrin. These fibrin degradation products include multimers such as DD fractions, DD/E fractions, DXD/YY fractions (trimers of DD/E fractions), YXY/DXXD fractions (pentamers of DD/E fractions) and DXXD/YXXY fractions (heptamers of DD/E fractions), and in particular, include large amounts of DXD/YY fractions (trimers of DD/E fractions), YXY/DXXD fractions (pentamers of DD/E fractions) and DXXD/YXXY fractions (heptamers of DD/E fractions) . Therefore, the reference material for measuring D-dimer, according to the present embodiment, contains fibrin degradation products having a molecular weight of no less than 500, 000 at a percentage of no less than 70 %, preferably no less than 75 %, and more preferably, no less than 80 % of the total amount of the fibrin degradation products. Here, the molecular weight of a fibrin degradation product can be measured in the molecular weight analysis by means of gel filtration chromatography, and as for the reference material for measuring D-dimer the area in the distribution chart that is occupied by fibrin degradation products having a molecular weight of no less than 500, 000 is no less than 70 %, preferably no less than 75 %, and more preferably, no less than 80 % of the total peak area.

Though DXD/YY fractions, YXY/DXXD fractions and DXXD/YXXY fractions, for example, can be cited as the fibrin degradation products having a molecular weight of no less than 500, 000, the fibrin degradation products are not limited to these. In addition, there is no particular upper limit in the molecular weight of these fibrin degradation products, and in general, the greatest molecular weight is approximately two million.

The conditions for the above described molecular weight analysis by means of gel filtration chromatography are as follows.
Column: Sephacryl S-300HR (made by Amersham Pharmacia Biotech Corporation), 1.6 x 70 cm
Eluate: 50 mM Tris-acetic acid buffer solution containing 0.1 M of NaCl, pH 7.1
Flow Rate: 0.375 mL/min
Detection Wavelength: 280 nm
Fraction Volume: 1.5 mL
Peak Area Calculation: Peak Fit software

The reference material for measuring D-dimer, according to the present embodiment, can be gained in accordance with a method that includes the step of making enzymes, which can degrade fibrin, act on fibrin (hereinafter, referred to as degradation step), and the step of fractionating the gained fibrin degradation products using gel filtration chromatography (hereinafter, referred to as fractionation step).

As for the fibrin that is used as the material, commercially available fibrin may be used or fibrin may be prepared from fibrinogen. In addition, fibrin that has been prepared from amino acid sequence of fibrin in accordance with a genetic engineering technique can be used. As for the method for preparing fibrin from fibrinogen, a method for making thrombin, an XIII factor and calcium salt act on a solution that includes purified fibrinogen can be cited. The fibrin that is gained in accordance with this method is in gel form, and this can be used in the degradation step as it is, or the fibrin from which moisture is removed by means of a freeze-dry method or the like, and which is further crushed so as to be in powder form, can be used in the degradation step.

As for the enzymes that can degrade fibrin, plasmin, urokinase and elastase are preferable, and a combination of two or more types from among these can be used.

As for the mixture ratio of fibrin and enzymes in the degradation step, it is preferable to mix 1 mU to 100 mU of enzymes per 1 mg of fibrin, more preferable to mix 1 mU to 75 mU of enzymes, even more preferable to mix 5 mU to 50 mU of enzymes, and it is most preferable to mix 10 mU to 20 mU of enzymes.

In the degradation step, it is preferable for the time during which fibrin and the enzymes are made to react to be approximately 1 hour to 24 hours, and it is more preferable for it to be approximately 4 hours to 10 hours. The reaction temperature can be appropriately selected according to the temperature that is optimal for the used enzymes, and in the case of plasmin, urokinase and elastase, approximately 37 °C is preferable.

It is preferable for the degradation reaction to be stopped at the final stage of the above described degradation step. As for the method for stopping the degradation reaction, there are no particular limitations as long as it is a method for inhibiting the activity of the enzymes, and for example, a method for adding an enzyme inhibitor to the reaction system can be cited. As a preferable enzyme inhibitor, aprotinin can be cited.

In the fractionation step, there are no particular limitations in gel particles which are used for the gel filtration chromatography as long as they are conventionally used for the separation of protein, and those of which the exclusion limit is a molecular weight of 10⁴ to 10⁶ are preferable, and Sephacryl S-300HR (made by Amersham Pharmacia Biotech Corporation) is more preferable.

The elution solvent that is used for the gel filtration chromatography can be appropriately selected in accordance with the type of the column, and in the case of the above described Sephacryl S-300HR, a buffer solution of which the pH is approximately 6 to 9 can be used, and a Tris-acetic acid buffer solution can be cited as an example.

The gained eluate is fractionated into fractions of a constant amount with the absorbance of each fraction being measured with a wavelength of 280 nm, and the molecular weight of the protein that is included in the fraction is confirmed so that a fraction of a high molecular weight can be sampled, and thereby, a desired fibrin degradation product can be gained. The method for confirming the molecular weight of the protein is not particularly limited as long as it is a conventionally known method, and a non-degenerated SDS-polyacrylamide gel electrophoresis (SDS-PAGE) method and a gel filtration chromatography method can be cited as examples.

It is preferable for the yield of D-dimer in the gained fibrin degradation products to be no less than 30 %, it is more preferable for it to be no less than 40 %, and it is most preferable for it to be 50 %.

The reference material for measuring D-dimer, according to the present embodiment, can be used as the fibrin degradation products that have been gained, as described above, after the concentration thereof has been appropriately adjusted by using them as they are, diluting them with an appropriate diluent or condensing them. Furthermore, fibrin degradation products that have been gained as described above and then frozen and dried can be used by adding a buffer solution at the time of use.

The reference material for measuring D-dimer, according to the present embodiment, may include a buffer solution, a protein stabilizer (such as, for example, bovine serum albumen (BSA)), a pH adjustor, a preservative (such as, for example, sodium azide or an antibiotic) and the like.

It is preferable for the buffer solution to have a buffering effect in the range of pH 5 to 10, preferably pH 6 to 9, and a phosphoric acid buffer solution, an imidazole buffer solution, a triethanol amine-hydrochloric acid, and a Good buffer solution can be cited as examples. As for the Good buffer solution, a variety of buffer solutions such as MES, Bis-Tris, ADA, PIPES, Bis-Tris-Propane, ACES, MOPS, MOPSO, BES, TES, HEPES, HEPPS, Tricine, Tris, Bicine and TAPS can be cited. Tris is more preferable.

The reference material for measuring D-dimer, according to the present embodiment, can be used as either the reference material or the reagent for quality control in the measurement of D-dimer.

The method for measuring D-dimer is not particularly limited as long as this is a method that is conventionally used in the art, and a latex agglutination method, ELISA and the like can be cited.

### Embodiments

The use underlying the present invention is described in further detail by citing embodiments in the following.

In the embodiments, the following measuring methods were used.

### Protein Concentration Measurement

The protein concentration of the protein solution that has been diluted with a physiological solution of sodium chloride so as to be 100 µg/mL to 700 µg/mL through the measurement of the absorbance for a wavelength of 280 nm was measured using any of an absorbance method (coefficient 1.37, Beginners Handbook for the Purification of Modified Protein, Amersham Pharmacia Biotech Publisher), a Lowry method (kit made by Bio-Rad Laboratories, Inc; Lowry OH, Rosebrough NJ, Farr AL, Randall RJ. Protein measurement with the folin phenol reagent. J Biol Chem (1951) 193: 265-275) and a Bradford method (kit made by Bio-Rad Laboratories, Inc; Operation Method for DC Protein Assay, published by Japan Bio-Rad Laboratories). As for the reference product, a reference product originated from BSA (made by Bio-Rad Laboratories, Inc) was used.

### SDS-Polyacrylamide Gel Electrophoresis (SDS-PAGE)

5 % to 10 % of gradient polyacrylamide gel and a MINI-PROTEIN II electrophoresis apparatus (made by Bio-Rad Laboratories, Inc.) were used so as to carry out a measuring process under a non-reducing environment. The protein was dyed using a Coomassie Brilliant Blue (CBB) kit (made by Bio-Rad Laboratories, Inc). The ratio of the amount of protein having a high molecular weight to the amount of protein having a low molecular weight was calculated in an image analysis using PhotoCaptMW (ver. 99.03) software.

### Embodiment 1

### (1) Preparation of Fibrin

A fibrin lump was prepared from the initial material in the following procedure. A pure fibrinogen material (made by Enzyme Research Laboratories) was dissolved in 50 mM of TBS (50 mM of Tris, 0.15 mM of NaCl, pH 7.4) so that a solution of 5 mg/mL of fibrinogen was gained, and calcium chloride (final concentration 25 mM, made by Kishida Chemical Co., Ltd.) and anXIII factor (final concentration 10 µg/ml, Fibrogammin product) were added to the solution, and after that, human thrombin (final concentration 2U/ml, made by Mitsubishi Pharma Corporation) was added. This mixture was incubated at 37 °C for 18 hours so as to gain fibrin in gel form. The gained gel was washed for 1 hour with 50 mM of TBS (pH 7.4), and then, the moisture was removed, and after that, this was put into a glass flask in eggplant shape so as to be frozen, dried and crushed using a PE2049-type freeze and dry apparatus (made by Japan Vacuum Technology Corporation), and thus, a fibrin powder was gained.

### (2) Degradation Step

700 mg of the gained fibrin powder was put into a polypropylene container of which the volume was 50 mL, and then was dissolved in 35 mL of 50 mM of TBS (pH 7.4) which was added into the container so that the concentration of fibrin became 20 mg/mL. Plasmin (P4895, made by Sigma Corporation) was added to this solution (final concentration 10 mU/mL), which was stirred at 37 °C for 6 hours. Aprotinin (made by Bayer Corporation) was then added so that the final concentration became 1,000 U/mL, and after that, centrifugal separation, with 10,000 x g, was carried out on the solution at 9 °C for 15 minutes, and thus, the clear liquid above the sediment (coarse D-dimer fractions) was gained.

### (3) Fractionation Step

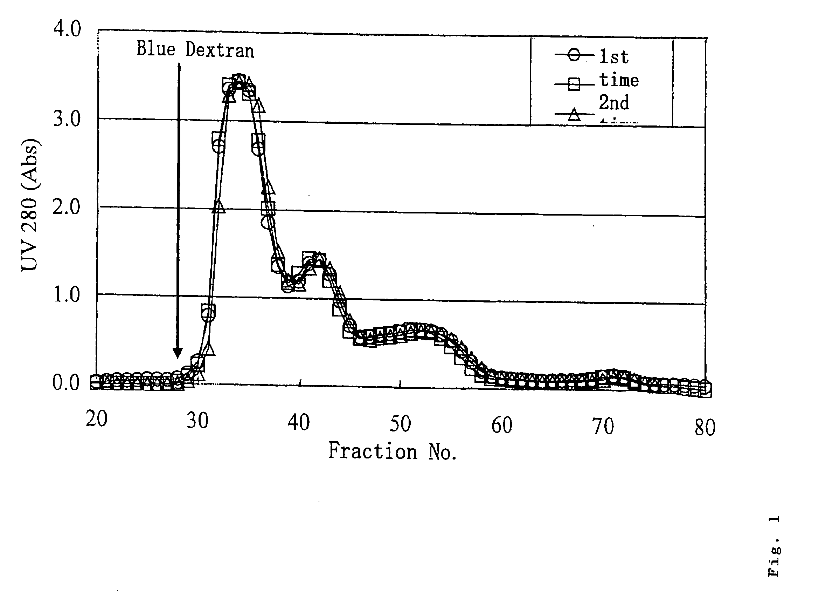

The coarse D-dimer fractions that had been gained in the above described degradation step were loaded into a Sephacryl S-300HR Column (1.6 x 70 cm, 120 mL, Amersham Pharmacia Biotech Corporation) where equilibrium was achieved with a Tris-acetic acid (pH 7.1) buffer liquid (flow rate 0.375 mL/min) of which the volume was 3 times as large as that of the column so that the amount of the protein became 80 mg. 50 mM of Tris-acetic acid (pH 7.1) buffer solution, which contained 100 KIU/mL of aprotinin and 0. 1 M of NaCl, was used as an eluate so that elution was carried out at a flow rate of 0.375 mL/min. Here, Blue Dextran (made by Amersham Pharmacia Biotech Corporation) was used as a void marker. The eluate was fractionated into amounts of 1.5 mL, and the absorbance of the gained fractions was measured for a wavelength of 280 nm. The results are shown in Fig. 1.

The relationships between the fractions and the molecular weights were checked using a protein of which the molecular weight was known, and then, it was found that protein of which the molecular weight was approximately 1 million eluted in fraction number 30, protein of which the molecular weight was approximately 500,000 eluted in fraction number 40, protein of which the molecular weight was approximately 200,000 eluted in fraction number 50, and protein of which the molecular weight was approximately 100,000 eluted in fraction number 60.

Here, the same procedure as the above described (1) to (3) was repeated three times so as to find that the charts, which were gained after the gel filtration column chromatography, all exhibited the same behavior (Fig. 1), and thus, it was confirmed that the above described procedure was reproducible.

After the elution of Blue Dextran, the fractions having the first to third peaks of absorbance for a wavelength of 280 nm (fractions 27 to 39 in Fig. 1) are collected and a reference material for measuring D-dimer was gained. The amount of protein of the gained reference material for measuring D-dimer was measured and was found to be 42.4 mg and the yield was 53 %.

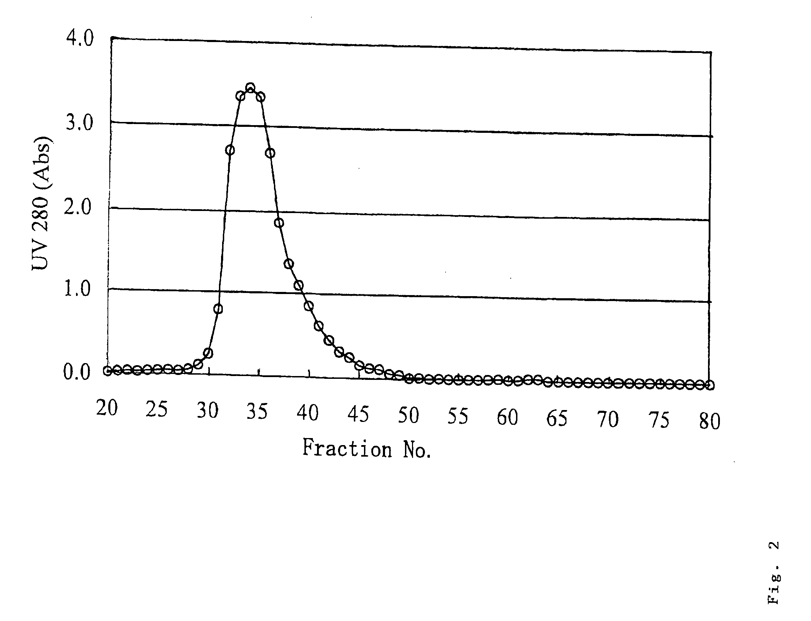

In addition, gel filtration chromatography was carried out on this reference material for measuring D-dimer under the same conditions as those for the fractionation step. The absorbance of the gained fractions for a wavelength of 280 nm was measured and the results are shown in Fig. 2.

The relationships between the molecular weight and the fractions were checked using protein of which the molecular weight was already known so as to find that protein of which the molecular weight was approximately 1 million eluted in fraction number 30, protein of which the molecular weight was approximately 500,000 eluted in fraction number 40 and protein of which the molecular weight was approximately 100,000 eluted in fraction number 60. Accordingly, the peak area up to fraction number 40 (peak area where the molecular weight is no less than approximately 500, 000) was calculated using peak fit software and was found to be 83 % of the total peak area.

### Test Example 1

The reference material for measuring D-dimer that has been gained in Embodiment 1 was diluted with a phosphoric acid buffer solution (pH 7.5) that contains 3 % (w/v) of BSA so that the following solutions are made in stages (1/1, 1/2, 1/4, 1/8, 1/16 and 1/32), and then the amount of D-dimer was measured using the following latex agglutination method. In the same manner, plasma of DIC patients (3 specimens) of which the concentrations are approximately 30 µg/mL was diluted in stages, and then, the amount D-dimer was measured using the following latex agglutination method.

### Method for Measuring Amount of D-dimer in Accordance with Latex Agglutination Method

First, latex particles, wherein a monoclonal antibody which recognizes D-dimer was sensitized, were prepared. 0.5 mL of a suspension of 10 % (w/v) polystyrene latex (made by Sekisui Chemical Co. , Ltd. , the diameter of particles 0.245 µm) was added to 2.0 mL of a phosphoric acid buffer liquid (pH 7.5) that contains a DD-M1653 antibody, which was produced using hybridomer cells which was entrusted with the National Institute of Advanced Industrial Science and Technology as trust number FERM P-19867, and which is a monoclonal antibody that recognizes D-dimer (antibody concentration 0.625 mg/mL), to be mixed by means of a vortex mixer. Centrifugal separation (25,000 xg, for 20 minutes) was carried out on this liquid mixture, which was then suspended in 2.5 mL of an MOPSO buffer solution (pH 7.1) which includes 1 % (w/v) of BSA. Centrifugal separation (25, 000 x g, for 20 minutes) was carried out on this suspension, which was then suspended in 40 mL of an MOPSO buffer solution (pH 7. 1) which includes 2 % (w/v) of BSA, and then a reagent that contains latex particles, where a DD-M1653 antibody was solidified, was gained.

14 µL of solution where the reference material for measuring D-dimer according to the present embodiment was diluted with a phosphoric acid buffer solution (pH 7.5) that contains 3 % (w/v) of BSA and 14 µL of a phosphoric acid buffer solution (pH 7.5) that contains 3 % (w/v) of BSA as negative control were mixed with 84 µL of an MOPSO buffer solution (pH 7.1) that contains polyethylene glycol so that a reaction occurs at 37 °C for 5 minutes. This is added to 84 µL of the above described reagent which contains latex particles that combine a monoclonal antibody, and the amount of change in the absorbance for a wavelength of 800 nm per minute was measured.

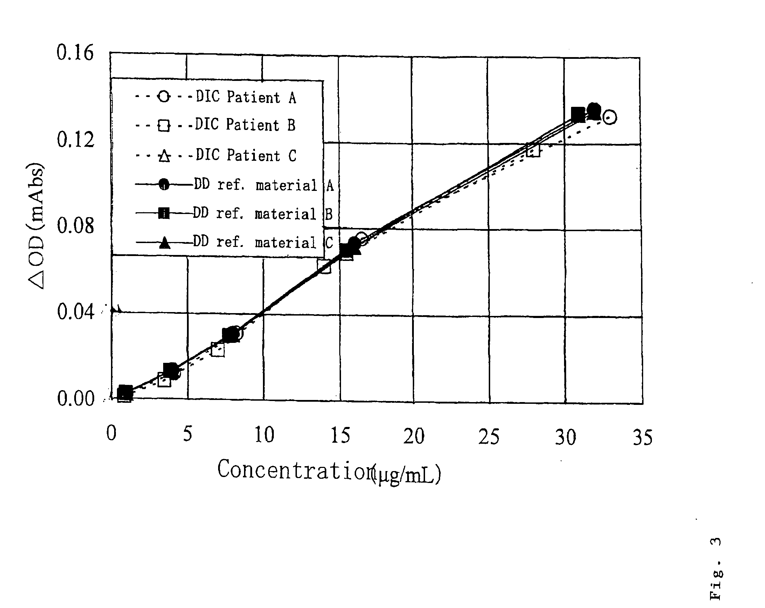

The results are shown in Fig. 3.

The reagents which were used in Fig. 3 as well as the concentrations of these before dilution were as follows.
Specimens from DIC patients: DIC patient A: concentration 33 µg/ml; DIC patient B: concentration 28 µg/ml; DIC patient C: concentration 31 µg/ml;
Reference material for measuring D-dimer according to the present embodiment: DD reference material A: concentration 32 µg/ml; DD reference material B: concentration 31 µg/ml; DD reference material C: concentration 32 µg/ml.

It was found from the results of Fig. 3 that the reference material for measuring D-dimer according to the present embodiment could be used for measuring the amount of D-dimer in accordance with a latex agglutination method and a calibration curve in accordance with the dilution ratio could be gained. Furthermore, it was found that the reference material for measuring D-dimer according to the present embodiment exhibits the behavior of the calibration curve that is the same as that of the results gained from plasma of the DIC patients.

Here, the same results were gained in accordance with a latex agglutination method for the respective reference materials for measuring D-dimer of three different lots which were gained by repeating the procedure of embodiment 1 three times (DD reference materials A, B and C) (Fig. 3).

### Test Example 2

### Comparison in Electrophoresis Patterns between Reference Material for Measuring D-dimer According to Present Embodiment and D-dimer Fraction in Plasma of Thrombosis Patient

The reference material for measuring D-dimer which was gained in Embodiment 1 and a sample that was gained by extracting serum from plasma of a patient, who was diagnosed with thrombosis, by means of a clot separator (made by Sysmex Corporation) were used, and these were diluted four times with a 1 % (w/v) solution of SDS and were spread out over 7.5 % of polyacrylamide gel which contains 0.1 % (w/v) of SDS under a constant voltage of 60 volts for 2.5 hours. As for the buffer solution, a 25 mM of Tris-192mM of glycine buffer solution (pH 7.5) which contains 0.02 % (w/v) of SDS was used.

This gel was made to make contact with a PVDF (polyvinylidene fluoride) membrane (made by Amersham Pharmacia Biotech Corporation), through which a current of 1 mA/cm² was made to flow for 3 hours so that the protein was transferred to the membrane. After that, the membrane was immersed in a blocking liquid (PBS (137 mM of NaCl, 2.7 mM of KCl, 1.5 mM of KH₂PO₄ , 8.1 mM of Na₂HPO₄) which contains 5 % of skim milk, 1 % (w/v) of BSA and 0.1% (w/v) of sodium azide) and was shaken for 30 minutes. The blocking liquid was discarded and the membrane was washed with PBS (washing liquid) which contains 0.05 % (w/v) of Tween20 for 5 minutes, three times. The membrane was immersed in PBS that contains 100 µg/mL of rabbit anti-fibrinogen polyclonal antibody (made by Dakosite Metly Corporation) and 0.05 % (w/v) of Tween20 as a primary antibody and was shaken for 60 minutes. The membrane was washed with a washing liquid for 5 minutes, 3 times and after that, was immersed in PBS that includes 100 µg/mL of HRP (horseradish peroxidase) labeled goat anti-rabbit antibody (made by Dakosite Metly Corporation) and 0.05 % (w/v) of Tween20 as a secondary antibody, and was shaken for 60 minutes. The membrane was washed with a washing liquid for 5 minutes, three times and after that, the band of protein was visualized using an HRP labeled kit (made by Bio-Rad Laboratories, Inc) with 4-chloro-1-naphthol and hydrogen peroxide.

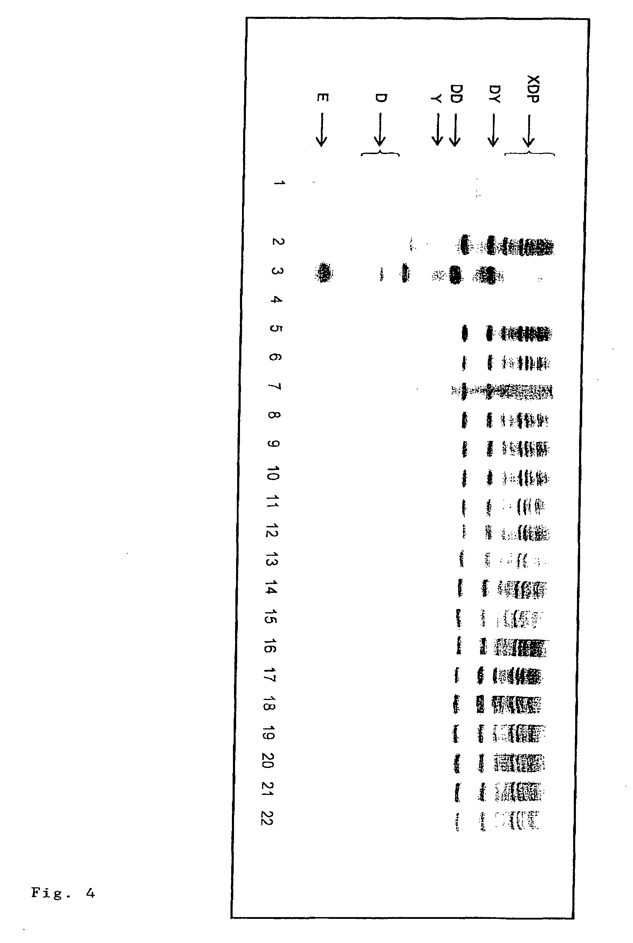

The results are shown in Fig. 4.

The specimens shown in Fig. 4 are as follows: lane 1 : molecular weight marker; lane 2: reference material for measuring D-dimer according to the present embodiment; lane 3: X fractions, Y fractions, D fractions, E fractions and DD fractions from among fibrin degradation products (made by Morinaga Biochemical Laboratories Corporation); lanes 5 to 22: specimens from thrombosis patients.

It can be seen from the results of Fig. 4 that the reference material for measuring D-dimer according to the present embodiment shows the distribution of D-dimer which closely resembles those of the specimens from the thrombosis patients.

## Claims

1. Use of a reference material as a reagent for quality control or calibration in assaying D-dimer, wherein said reference material comprises a fraction of a fibrin degradation product, wherein no less than 70 % of the total amount of fibrin degradation products in said fraction has a molecular weight of no less than 500,000.

2. Use of a reference material according to claim 1, wherein said reference material further comprises a buffer solution.

3. Use of a reference material according to claim 2, wherein said buffer solution has a pH of 5 to 10.

4. Use of a reference material according to any of claims 1 to 3, wherein said reference material further comprises a protein stabilizer.

5. Use of a reference material according to claim 4, wherein said protein stabilizer is BSA.

6. Use of a reference material according to any of claims 1 to 5, wherein no less than 75 % of the total amount of fibrin degradation products in said fraction has the molecular weight of no less than 500,000.

7. Use of a reference material according to any of claims 1 to 6, wherein no less than 80 % of the total amount of fibrin degradation products in said fraction has the molecular weight of no less than 500,000.

8. Use of a reference material according to any of claims 1 to 7, wherein the fibrin degradation product is prepared by using a fibrin degrading enzyme.

9. Use of a reference material according to claim 8, wherein said fibrin degrading enzyme is selected from the group consisting of plasmin, urokinase and elastase.

10. Use of a reference material according to claim 1, wherein no less than 70 % of the total amount of fibrin degradation products in said fraction has the molecular weight of no less than 500,000 and less than 2000,000.

11. Use of a reference material according to claim 6, wherein no less than 75 % of the total amount of fibrin degradation products in said fraction has the molecular weight of no less than 500,000 and less than 2000,000.

12. Use of a reference material according to claim 7, wherein no less than 80 % of the total amount of fibrin degradation products in said fraction has the molecular weight of no less than 500,000 and less than 2000,000.

## Patentansprüche

1. Verwendung eines Referenzmaterials als Reagens für die Qualitätskontrolle oder Kalibrierung bei der Untersuchung eines D-Dimers, wobei das Referenzmaterial eine Fraktion eines Fibrinabbauprodukts enthält, wobei nicht weniger als 70% der Gesamtmenge an Fibrinabbauprodukten in der Fraktion ein Molekulargewicht von nicht weniger als 500 000 aufweisen.

2. Verwendung eines Referenzmaterials nach Anspruch 1, wobei das Referenzmaterial weiterhin eine Pufferlösung umfasst.

3. Verwendung eines Referenzmaterials nach Anspruch 2, wobei die Pufferlösung einen pH-Wert von 5 bis 10 aufweist.

4. Verwendung eines Referenzmaterials nach einem der Ansprüche 1 bis 3, wobei das Referenzmaterial weiterhin einen Proteinstabilisator umfasst.

5. Verwendung eines Referenzmaterials nach Anspruch 4, wobei es sich bei dem Proteinstabilisator um BSA handelt.

6. Verwendung eines Referenzmaterials nach einem der Ansprüche 1 bis 5, wobei nicht weniger als 75% der Gesamtmenge an Fibrinabbauprodukten in der Fraktion ein Molekulargewicht von nicht weniger als 500 000 aufweisen.

7. Verwendung eines Referenzmaterials nach einem der Ansprüche 1 bis 6, wobei nicht weniger als 80% der Gesamtmenge an Fibrinabbauprodukten in der Fraktion ein Molekulargewicht von nicht weniger als 500 000 aufweisen.

8. Verwendung eines Referenzmaterials nach einem der Ansprüche 1 bis 7, wobei das Fibrinabbauprodukt durch Verwendung eines fibrinabbauenden Enzyms hergestellt wird.

9. Verwendung eines Referenzmaterials nach Anspruch 8, wobei das fibrinabbauende Enzym aus der Gruppe bestehend aus Plasmin, Urokinase und Elastase ausgewählt ist.

10. Verwendung eines Referenzmaterials nach Anspruch 1, wobei nicht weniger als 70% der Gesamtmenge an Fibrinabbauprodukten in der Fraktion ein Molekulargewicht von nicht weniger als 500 000 und weniger als 2 000 000 aufweisen.

11. Verwendung eines Referenzmaterials nach Anspruch 6, wobei nicht weniger als 75% der Gesamtmenge an Fibrinabbauprodukten in der Fraktion ein Molekulargewicht von nicht weniger als 500 000 und weniger als 2 000 000 aufweisen.

12. Verwendung eines Referenzmaterials nach Anspruch 7, wobei nicht weniger als 80% der Gesamtmenge an Fibrinabbauprodukten in der Fraktion ein Molekulargewicht von nicht weniger als 500 000 und weniger als 2 000 000 aufweisen.

## Revendications

1. Utilisation d'un matériau de référence en tant que réactif pour le contrôle qualité ou l'étalonnage dans un dosage de D-dimère, ledit matériau de référence comprenant une fraction d'un produit de dégradation de fibrine, dans lequel pas moins de 70 % de la quantité totale de produits de dégradation de fibrine dans ladite fraction a un poids moléculaire de pas moins de 500 000.

2. Utilisation d'un matériau de référence selon la revendication 1, ledit matériau de référence comprenant en outre une solution tampon.

3. Utilisation d'un matériau de référence selon la revendication 2, ladite solution tampon ayant un pH de 5 à 10.

4. Utilisation d'un matériau de référence selon l'une quelconque des revendications 1 à 3, ledit matériau de référence comprenant en outre un stabilisant protéique.

5. Utilisation d'un matériau de référence selon la revendication 4, ledit stabilisant protéique étant BSA.

6. Utilisation d'un matériau de référence selon l'une quelconque des revendications 1 à 5, pas moins de 75 % de la quantité totale de produits de dégradation de fibrine dans ladite fraction ayant un poids moléculaire de pas moins de 500 000.

7. Utilisation d'un matériau de référence selon l'une quelconque des revendications 1 à 6, pas moins de 80 % de la quantité totale de produits de dégradation de fibrine dans ladite fraction ayant un poids moléculaire de pas moins de 500 000.

8. Utilisation d'un matériau de référence selon l'une quelconque des revendications 1 à 7, le produit de dégradation de fibrine étant préparé au moyen d'une enzyme dégradant la fibrine.

9. Utilisation d'un matériau de référence selon la revendication 8, ladite enzyme dégradant la fibrine étant choisie dans le groupe constitué de la plasmine, l'urokinase et l'élastase.

10. Utilisation d'un matériau de référence selon la revendication 1, pas moins de 70 % de la quantité totale de produits de dégradation de fibrine dans ladite fraction ayant un poids moléculaire de pas moins de 500 000 et inférieur à 2 000 000.

11. Utilisation d'un matériau de référence selon la revendication 6, pas moins de 75 % de la quantité totale de produits de dégradation de fibrine dans ladite fraction ayant un poids moléculaire de pas moins de 500 000 et inférieur à 2 000 000.

12. Utilisation d'un matériau de référence selon la revendication 7, pas moins de 80 % de la quantité totale de produits de dégradation de fibrine dans ladite fraction ayant un poids moléculaire de pas moins de 500 000 et inférieur à 2 000 000.
